# EUROPEAN PATENT SPECIFICATION

(11) **EP 4 188 480 B1**
(45) Date of publication and mention of the grant of the patent: **21.10.2026**
(21) Application number: 21743516.3
(22) Date of filing: 16.07.2021
(51) Int. Cl.: A61M 1/36, A61M 5/165, A61M 5/38, B01D 19/00, A61M 5/36

(54) **INFUSION APPARATUS**
INFUSIONSVORRICHTUNG
APPAREIL DE PERFUSION

(30) Priority: 29.07.2020 EP 20188400
(43) Date of publication of application: 07.06.2023
(73) Proprietor: Tessen Solutions Limited, P43 T299 Cork (IE)
(72) Inventor: FORDE, Vincent, Cork Institute of Technology, Bishopstown Cork, T12 Y275 (IE)
(74) Representative: Weldon O'Brien Ltd.
(86) International application number: PCT/EP2021/069973
(87) International publication number: WO 2022/023079

(56) References cited:
- EP-A1- 0 423 841
- WO-A1-2005/053772
- WO-A2-2011/128850
- GB-A- 2 059 776
- US-A- 4 048 995

## Description

### Introduction

This invention relates to a bubble trap apparatus suitable for medical applications, especially infusion with primary and secondary lines of infusion fluids.

GB2059776 describes an intravenous administration set for a primary liquid and a secondary liquid. US4,048,995 describes an injection site with parallel first and second inlets. WO2005/053772 describes a degassing device and end cap assembly. WO2011/128850 describes an intravenous pump with a bubble detector and an air trap module. EP0423841 describes a venous reservoir with a blood collecting chamber.

During intravenous (IV) infusion natural degassing can occur, particularly infusions of two or more chemically incompatible fluids, leading to bubbles of gas that can pose a threat to the health of a patient. Where toxic biologics or hazardous drugs are used, such as in Chemotherapy, the gases released can also be toxic and should be retained within a safe chamber. In particular, development of a Closed System Aspirator (CSA) would enhance the current state of the art and improve safety during healthcare practice.

IV infusion of drugs is common in modern healthcare. The clinical action of combining fluid drugs for infusion directly into the vein is standard practice, especially in the field of Oncology where expensive or highly targeted drug doses are required. In this case, the drug volume is critical and fluid losses due to adhesion to IV tubing are not tolerated. The primary fluid, often Normal Saline (NS) or Ringer's Lactate, is used as a carrier fluid to flush the maximum amount of the drug into the patient's vascular system. The secondary fluid can include a broad range of biologics, drugs or hazardous drugs (HD) such as Etoposide^{™} or Paclitaxel^{™}. Some drugs are contraindicated to particular tubing types, such as PVC tubing, due to sidewall adhesion and benefit from the lower friction properties when mixed as a solution with NS.

A chemical reaction can occur when two or more liquid drugs are combined into a mixture. As a result of chemical reactions the drugs may elute or degas. This degassing can also occur due to turbulent reactions between the liquid molecules. Turbulence occurs due to friction responses as fluids move across surfaces and current eddies forming as molecules of liquid rapidly change direction and interact due to changes of the surrounding geometry.

The fluid in these situations is a combination of liquid, gas and plasma into a single phase. Bubbles of gas are released in the fluid when fluids degas. Bubbles form as pressure of the gas pushes outwards from inside, while the surrounding water molecules tend to stick together and form a membrane. The greater the pressure from the gas on the bubble membrane, the larger the bubble that forms. Where bubbles interact the bubble membranes can coalesce and combine to create larger bubbles. In a body of fluids, the bubble is acted upon by the surrounding forces of pressure and tend to float upwards under buoyancy forces.

In use, certain mixtures or drugs are gassy and unstable due to their chemical structure. These liquids have a tendency to give off volumes of gas that require a responsive action from healthcare professionals to reduce the risk for the patient. One of these important actions is removal of the bubble from the liquid. In the case where the bubble is formed from toxic biologics or hazardous drugs the resulting vapour has properties that may be hazardous to human health. Contact with these drug vapours should be avoided.

It is known to provide a 'piggyback' IV drip set-up illustrating various connection devices and techniques for infusing additional drugs into a primary line. A commonly used setup is to have maintenance fluids, such as Normal Saline or Lactated Ringer's, infusing as the primary fluid. Chemotherapy may be administered through a secondary port or Y-port. A secondary fluid is infused into the primary fluid, sometimes known as 'piggybacking'. The secondary IV solutions bag is hung above the height of the primary bag. Position of the IV solutions influences the flow of the IV fluid into the patient. The setup is the same if the medication is given by gravity or through an IV infusion pump. The setup of equipment and the ratio of the mixture of the two fluids can have an effect on flow rate and dosing accuracy.

Up to 75% of chemotherapy is delivered intravenously in the form of hazardous drugs (HD) that are antineoplastic, mutagenic, carcinogenic and harmful for human health. Intravenously administered drugs can be classified into five categories according to their damage potential: Vesicant, Exfoliants, Irritants, Inflammitants, and Neutrals. Closed system devices are used to minimise exposure and related adverse effects for medical staff that handle these HD. Closed System Transfer Devices (CSTD) are defined as "a drug transfer device that mechanically prohibits the transfer of environmental contaminants into the system and the escape of the hazardous drug or vapor concentrations outside the system" [NIOSH 2004]. While many forms of CSTD exist to transfer drugs into an IV system, there remains a need to remove the problem bubbles efficiently while still protecting the surrounding environment from exposure.

Administering multiple intravenous (IV) infusions to a single patient via infusion pump occurs routinely in health care. Interruptions to pump function occur when problem air bubbles form in the IV tube. Modern IV pumps use sensor technology to observe bubbles in the line and stop the infusion, which can lead to dosing errors, inefficiency and loss of drug effectiveness where short half-life is a drug feature. Micro-bubbles are small bubbles with a diameter between 10 to 50µm and decreasing in size and lastly disappear under water. Ordinary bubbles have a diameter which range from 1µm and larger. The removal of ordinary and micro-bubbles from IV lines would contribute to an improvement in drug delivery, accuracy, safety and healthcare time efficiencies. Bubbles in IV infusions typically sound an alarm on the infusion pump device, creating noise pollution, a leading cause of alarm fatigue among healthcare workers. As such, removing these naturally forming bubbles before they reach the IV pump can have beneficial effects on healthcare performance.

When joining two infusion lines together, normally a Y-connector device is used. This device can be in the form of a rubber bung and spike, or a male Luer to female Luer connection. Where HD are used it more common and preferable to use Luer-Luer to minimise risk of disconnection or improper sealing between components that enables vapour to be expelled to the atmosphere. Many hospitals and health facilities prepare HD in the controlled pharmacy section where adequate ventilation is installed, however the action of removing problem bubbles occurs at the bedside. The attending nurse will typically use a syringe and manual manipulation technique to tap or flick the IV line, displacing the bubble into a junction where it can be manually removed by drawing the bubble and a small quantity of fluids into a syringe. This requires close contact with the HD vapour, potential risk of spillage and wastage of the critical drug volume. The actions required to remove a bubble are most often undertaken by nursing staff using improper tools and non-standardised techniques which can place the nurse at risk of exposure and harm.

The invention addresses the above problems.

### Summary of the Description

We describe a gas trap apparatus as set out in claim 1 appended hereto, and various aspects of apparatus' as set out in claims 2 to 15.

### Detailed Description of the Invention

The invention will be more clearly understood from the following description of some embodiments thereof, given by way of example only with reference to the accompanying drawings in which:
Fig. 1 is view of an infusion system incorporating a trap at a junction of primary and secondary lines;
Fig. 2 is a diagrammatic side view of the trap of Fig. 1, arranged primarily for laminar entry;
Fig. 3 is a diagrammatic side view of a trap arranged primarily for turbulent entry;
Fig. 4 is a diagrammatic side view of a trap arranged primarily for using the secondary port to vent the primary fluids;
Fig. 5 is a diagrammatic side view showing a trap a dedicated vent port to vent the combined primary and secondary fluids solution;
Fig. 6 is a diagrammatic side view of an inlet port assembly for primary and secondary infusion ports;
Fig. 7 is a similar view of an inlet port assembly with three inlet ports and a selectively sealable membrane;
Figs. 8(a), (b), an (c) are views of a trap incorporating a hydrophilic membrane material to capture and retain artefacts;
Figs. 9(a) and (b) are views of a trap with a number of inlet ports in a staggered arrangement;
Fig. 10 is a set of views of traps with arrangements to fix its position relative to an IV pole or to be fixed;
Figs. 11(a), (b), (c), and (d) are views of traps with arrangements that include a valve to control flow between primary and secondary ports, as well as flow into the chamber for each port,
Fig. 12 (a) and 12(b) re views of a trap apparatus with a manifold for j oining multiple lines;
Fig. 13 is a diagram showing a port and its sealing arrangement; Fig. 14 shows an alternative port cap with a more positive sealing engagement; and Figs 15 and 16 are plan views of further alternative port caps, in this case with notches for engagement by a tool;
Figs. 17 to 19 are perspective views showing an alternative port, in this case with the cap being linked by a ribbon;
Fig. 20 is a set of side and sectional views of an alternative port, in this case with Luer threads; and
Figs. 21 and 22 are side views of alternative ports, in this case with threads and with connector ribbons.

We describe an infusion apparatus and a gas/bubble trap apparatus for an infusion apparatus. The trap has two or more inlets for liquid, and it retains bubbles and resultant gas in the trap in a manner which is safe for medical personnel as it prevents escape of gases which are undesirable in the ambient air. Also. in various examples it allows the gases to be removed safely, and in methods of operation it allows unsafe gases to be neutralized.

The apparatus is suitable for infusing primary and secondary drugs individually or in combination and collecting gas bubbles entrained in a liquid within a sealed chamber. There is a housing defining at least one chamber, at least one inlet port, and an outlet port. For mixing inlet fluids there are preferably multiple inlet ports.

The bubble traps have a chamber which is in some examples spherical. An inflow tube delivers fluid in a manner to deflect radially by a diverter which is preferably conical in shape. The outflow tube has an inlet which is downstream of the diverter and so is very unlikely to receive any bubbles which are deflected by the diverter. The inflow tube preferably has a diffuser upstream of the diverter, such as a ring of apertures around its circumference. Liquid and gases will diffuse out through the diffuser, which tends to constrict the flow, resulting in a more controlled flow laterally from the diffuser causing any bubbles to congregate against an internal surface of the chamber and away from the outflow tube inlet.

In general terms the apparatus has a housing defining at least one chamber, the chamber having an inlet and an outlet; a diverter in the chamber positioned between the inlet and the outlet. The outlet is preferably provided by an outflow tube with an inlet end within the chamber. The inlet provides flow in a longitudinal direction into the chamber, and the diverter diverts flow laterally with respect to this direction. The outlet is preferably aligned with the inlet, or it may be at an angle to it. The outflow tube inlet is located within the chamber. The diverter preferably comprises a base portion and a rim, and it preferably defines a downstream-facing volume into which it is unlikely that bubbles will enter. The outflow tube inlet is preferably located within a central volume defined by a range within 40% to 60% across any dimension across the chamber.

In various examples:
a portion of the inflow tube abuts the diverter or is integral with the diverter;
the diverter has rounded contours;
the apparatus comprises at least one venting port suitable for purging trapped gas bubbles from the apparatus;
there is at least one venting port suitable for purging trapped gas bubbles from the apparatus;
there may be a venting port coupled with a release means, the release means being operable to move between a closed position to an open position; such that, in use, when the release means is in the open position the trapped gas is purged from the chamber of the apparatus through the venting port;
at least one venting port is suitable for purging trapped gas bubbles from the apparatus;
the release means is automated;
the venting port comprises a gas permeable-water impermeable membrane such that, in use, trapped gas traverses the gas pervious/water impervious membrane and is purged from the chamber, while water is retained within the chamber of the apparatus;
the chamber is spherical;
the apparatus is arranged in a circuit or infusion system for delivery to a patient;
the apparatus comprises a plurality of chambers arranged in a series;
the chamber and the diverter have an internal surface which has a surface treatment for hydrophilic control of a bubble and its movement within the chamber;
the rim of the diverter surrounds the intake end of the exit tube;
the apparatus comprises a gas-impermeable and liquid-permeable membrane at the intake end of the exit tube;
the apparatus comprises a filter to remove particulates before entry of liquid to the intake end of the exit tube;

We also describe an intravenous line kit comprising at least one intravenous drip bag, at least one drip chamber; at least one supply tube having a proximal end and a distal end; a bubble trap; a fluid flow control means; at least one clamp; and a cannula; wherein the bubble trap comprises:
a housing defining at least one chamber, the chamber having an inlet port and an outlet port;
an inflow tube with an intake end and an export end, the export end being located within the chamber;
a diverter positioned between the chamber inlet port and the chamber outlet port, wherein the diverter comprises a base portion and a rim;
an exit tube with an intake end and an export end, wherein said intake end of the exit tube is located within the chamber and said export end of the exit tube is connected to the outlet port of the chamber; and
a diffuser in the inflow tube between the inlet port and the diverter, wherein the diffuser comprises at least one hole in the inflow tube, and is configured to impart an alternative direction of movement of fluid entering the chamber in the inflow tube.

The above features are combined in some examples with inflow conduits which provide for merging of different flows upstream of the chamber or within the chamber, but upstream of the diverter within the chamber, as described in more detail below.

The inlet preferably comprises a plurality of inlet ports for mixing of different fluids for optimum patient administration. The bubble trap has the major advantage of preventing bubbles from flowing out through the outlet, even if there are abnormally high levels of gas due to the mixing of fluids via the different ports.

There may be a venting port, which is preferably sealable with a cap. There may be a diffuser channel that separates the infusion fluids or actively combines the fluids to accelerate degassing. There may be a diffuser section that controls bubble behaviour as it enters the chamber. A diverter may be positioned between the inlet ports and the outlet port so that the direction of fluid flow can be controlled.

There may be a self-sealing membrane that enables fluids or gases to be pushed into or drawn from the chamber via one of the infusion ports or a venting port.

There may be an elongated exit tube that has an export end located in the centremost region of the chamber, the export end of which is connected to the outlet port of the chamber.

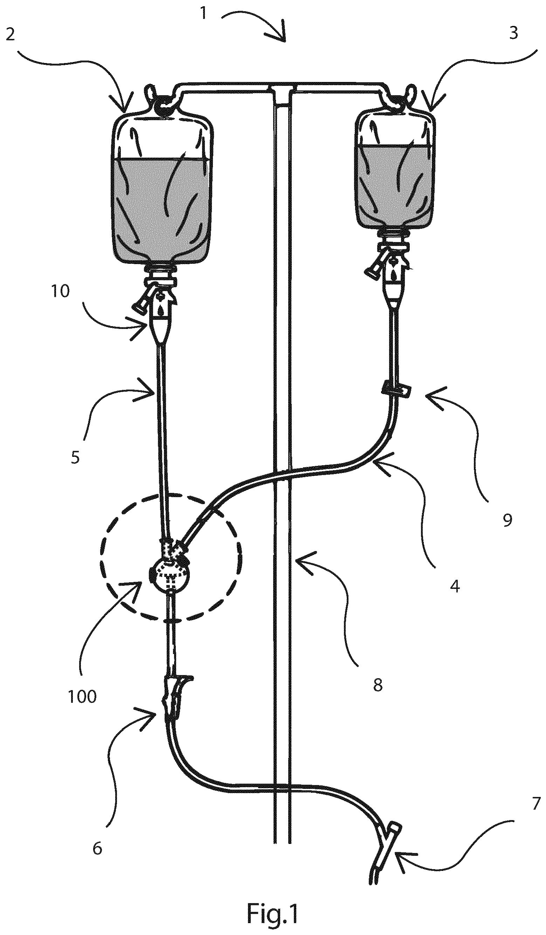

We also describe a closed-system aspirator apparatus with some or all of the above features. Referring to Fig. 1 a 'piggyback' IV drip set-up 1 has a primary fluids bag 2, a secondary fluids bag 3, a piggyback set 4, a primary set 5, a roller clamp 6, a Y-connector port 7, an IV pole 8, a clamp 9, a dropper 10, and a bubble trap apparatus 100. Fig. 1 illustrates various connection devices and techniques for infusing additional drugs. The chamber may be fully sealable so that it that can retain all fluids or gases. The bubble trap apparatus 100 is described below, but in general terms the apparatus may comprise any of the features described herein in various examples.

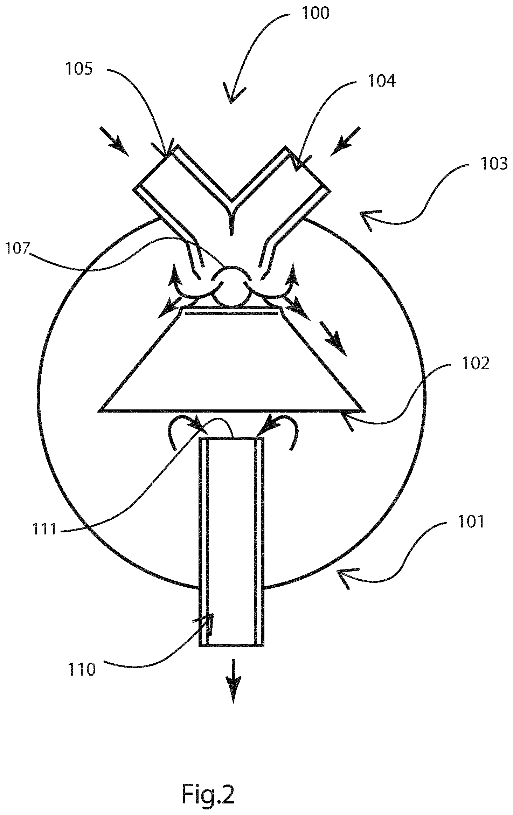

Referring to Fig. 2 the trap 100 has a spherical chamber 101 and an inlet with first and second inlet ports 104 and 105 leading to a diverter 102 of frusto-conical shape. This is arranged primarily to smooth fluid eddies and convert turbulent flow to laminar flow in the entry corridor. The arrows show the flows of gas bubbles upwardly from diffuser apertures 107 and flow of liquid into an inlet 111 of an outflow tube 110 and out through the outflow tube 110. This trap apparatus may be used to receive different fluids which mix in the chamber 101, and the bubbles which migrate upwardly can combine to be less harmful, and in any event, they are trapped in the chamber 101.

The trap 100 has the benefit of catering for many of the situations set out in the Introduction above, in which there is a greater risk of a gas being routed onwards to the patient. By having an inlet 103 in the form of a Y-type junction with two inlet ports, there is opportunity for fluids to mix immediately upstream of the chamber, and for the collision of fluid particles from two streams to accelerate degassing, mixing of fluids and concomitant phase separation. The gases which are conveyed or are created by this mixing are immediately diverted via the diffuser 107 in lateral directions towards the wall of the chamber 101 and away from the outflow tube inlet 111. Importantly, the diffuser 107 has the effect of slowing and redirecting the flow, thereby helping to ensure that the lateral flow is controlled in a manner that expedites separation via buoyancy effects, and helps to keep bubbles in the chamber near the internal chamber surface and away from the inlet 111. The space including and between the confluence of the multiple inlet ports, within and out through the diffuser, and adjacent the diffuser in the chamber is turbulent in use, promoting formation of bubbles from gas so that it efficiently rises and becomes trapped in the chamber. It is preferred that for inlet ports of round cross-section and diameters in the range of 1.5 to 8.0 mm the distance from the diffuser holes and the upper end of the inlet port confluence is no more than 20mm.

This arrangement induces and promotes turbulence as the fluids mix, and this turbulence continues through the diffuser, thereby accelerating degassing of the mixture, enhancing subsequent bubble formation and hence migration of the gas as bubbles away from the outlet, towards an upper end of the chamber in use.

As bubbles rise under pressures exerted from the fluid body, the chamber capacity is optimised to hold more or less than 10 millilitres of fluid in a fully primed state, with a relative ability to retain up to 50% of the volume in a gas state. The inlet has the advantage of providing a confluence for different fluids, positioned at a distance measuring equal at least to the internal diameter of the inflow tubes (104, 105), immediately upstream of the diffuser (107), useful for the common practice of combining and administering fluid drug formulas, thereby allowing immediate lateral outflow of the bubbles which are formed.

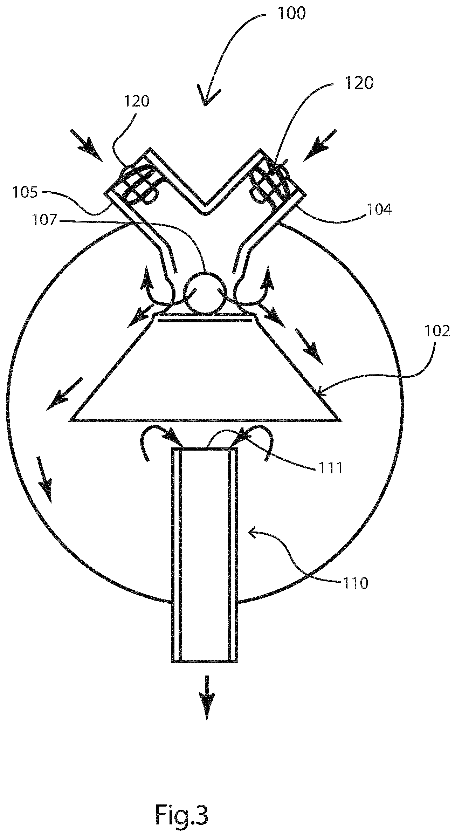

Fig. 3 shows the trap 100, arranged primarily for turbulent entry, with barriers 120 in a helical shape in the inlet ports 104 and 105. Like parts are indicated by the same reference numerals.

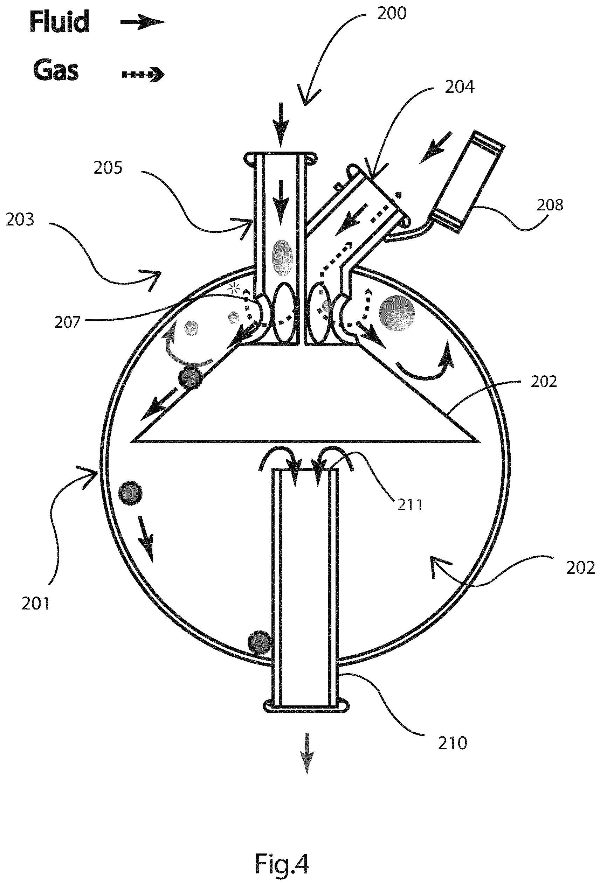

Referring to Fig. 4 a trap 200 is arranged primarily for using a secondary port to vent the primary fluids. It comprises a chamber 201, a diverter 202, an inlet 203 with inlet ports 204 and 205 and diffuser holes 207, and an outlet port 210 with an inlet 211. In this case the inlet port 204 is used to vent primary fluids delivered via the port 205. It is particularly advantageous for many examples of use that a second inlet port is left open so that it vents the gases which enter via the other inlet port. This arrangement is particularly suitable where gases can safely be vented to ambient, providing the flexibility of a second port and a vent. Where required the inlet port 204 can be capped or sealed with a selectively openable cap 208. In the event of the port 204 being required as an inlet for an additional fluid the cap 208 may be opened. In the scenario where the fluids are toxic, such as chemotherapy, the cap 208 may be indicated by colour or design as not suitable for reopening.

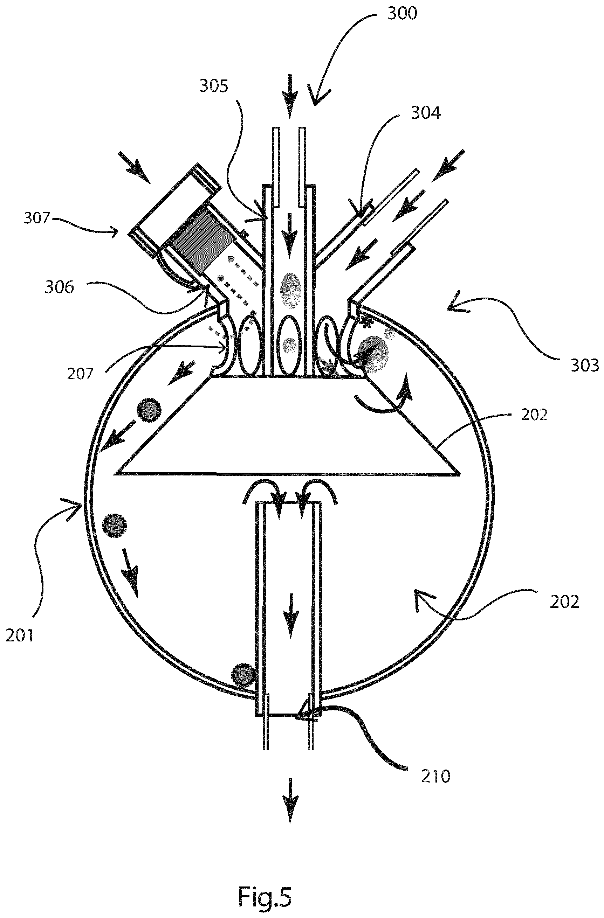

Referring to Fig. 5 a trap 300 has a chamber and a diverter of similar configuration, and an inlet 303 with three ports 304, 305, and 306. Two can be used for inflow of a fluid, while the third is used selectively as a dedicated venting port for safe removal to vent the combined primary and secondary fluids solution. The port 306 has a removable cap 307 for selective venting.

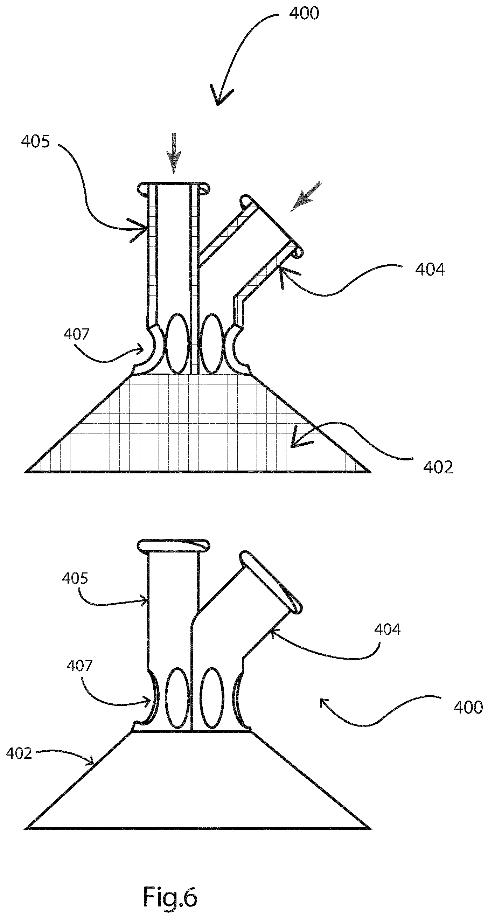

Fig. 6 shows an integral component 400 which is mounted during manufacture to a chamber. The component 400 has inlet ports 404 and 405 and a frusto-conical diverter 402, and diffuser openings 407 for inflow of fluids with a radial component into and around the diverter 402. An integral component such as this provides simple and accurate assembly.

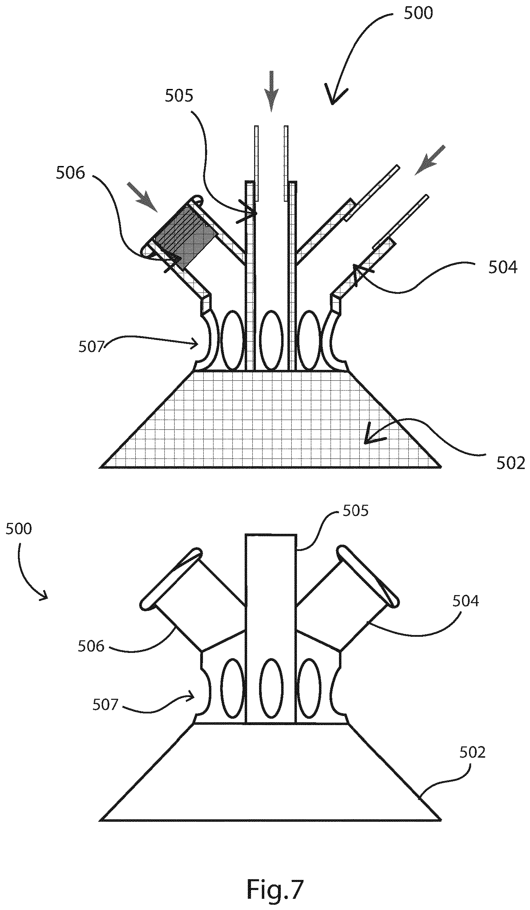

Referring to Fig. 7 an integral inlet port assembly 500 has primary and secondary infusion ports 504 and 505, and a third port 506 all converging at a diffuser 507 at the base of a diverter 502, and openings 505 in-between. This component has a selectively sealable membrane located in one or more of the infusion ports or venting ports 504-506. The membrane is activated by insertion of a compatible needleless connector device. The purpose of the normally sealed or self-healing membrane is to maintain the closed system integrity when venting toxic gas from the chamber into a selectively attached closed system transfer device with needless connectivity. The membrane also maintains a closed system for import of a drug directly into the chamber through a port via a closed system transfer device, normal syringe, tubing or any other suitable device. Such a membrane may be formed from rubber, silicone or other material that can elastically deform and return to normal shape. This membrane, which is normally closed but can deform to allow passage of fluids through a channel, may be acted upon by a needleless connector that forces the normally sealed membrane to deform and open its channel such that liquids can pass through. In such a case the liquids can be injected into the chamber or syphoned from the chamber by actuating the needleless syringe. Similarly, any other device or IV tubing with suitable needleless connector may be attached by means of a Luer connection or push fit or other connection. Once the needleless connector device is withdrawn the self-healing membrane, for example that marketed as B Braun's CARESITE^{®}, will return to its normally closed position to seal the port. The port may be further secured by closure of a tap or a cap that can be pushed, twisted or pressed in place, or any other method of sealing the end of the port.

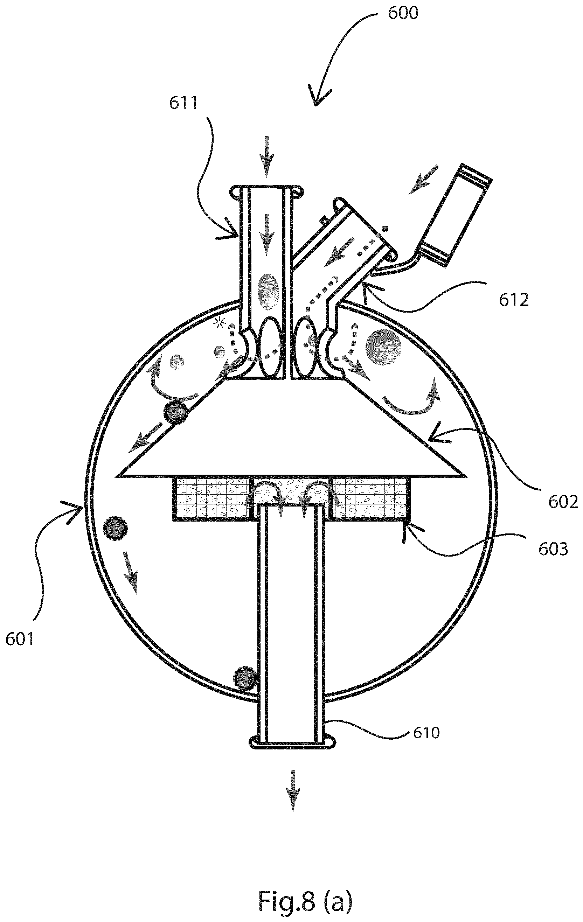

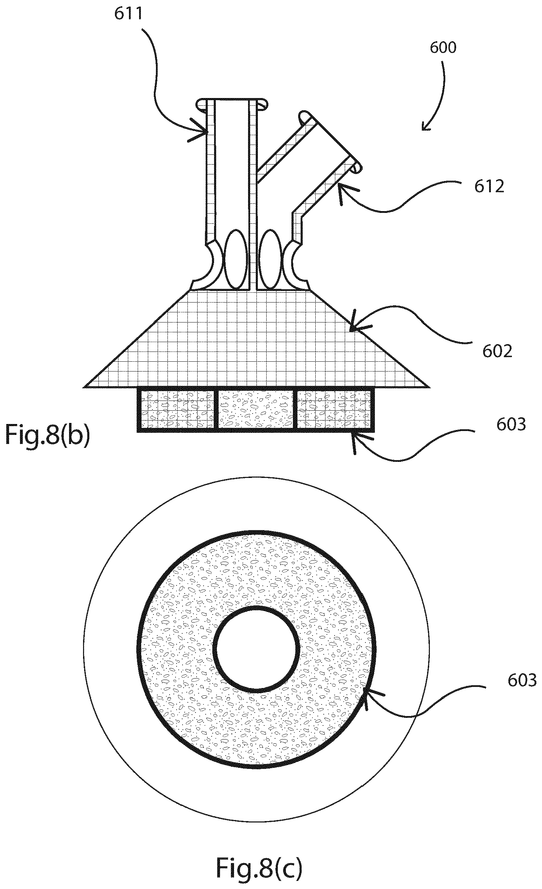

Referring to Figs. 8 (a), (b), an (c) a trap 600 has a spherical chamber 601, a conical diverter 602, an outflow tube 610, and inlet ports 611 and 612. The trap 600 also comprises a hydrophilic membrane 603 to capture and retain artefacts. It comprises a chamber 601, a diverter 602, and an inlet 600 having inlet ports 611 and 612. The membrane 603 is underneath the diverter 602 and it comprises a fine mesh of fibres, forming a fabric of material, such as PTFE, that have micron-level openings to allow only fluid particles to flow through while capturing solid or gas particles that are above the size of the mesh openings. Hydrophilic membrane materials are commonly used in healthcare to prevent particulates and artefacts of greater than 0.2 micron in size from transporting into the patient's bloodstream. In the shown embodiment the membrane attaches to the base of the conical diverter 602 and abuts the opening of the outflow tube 610 and has dimensions that ensure the gap between the diverter and outflow tube is sealed. In another embodiment the membrane may be stretched across the opening of the outflow tube 610 within the chamber 601.

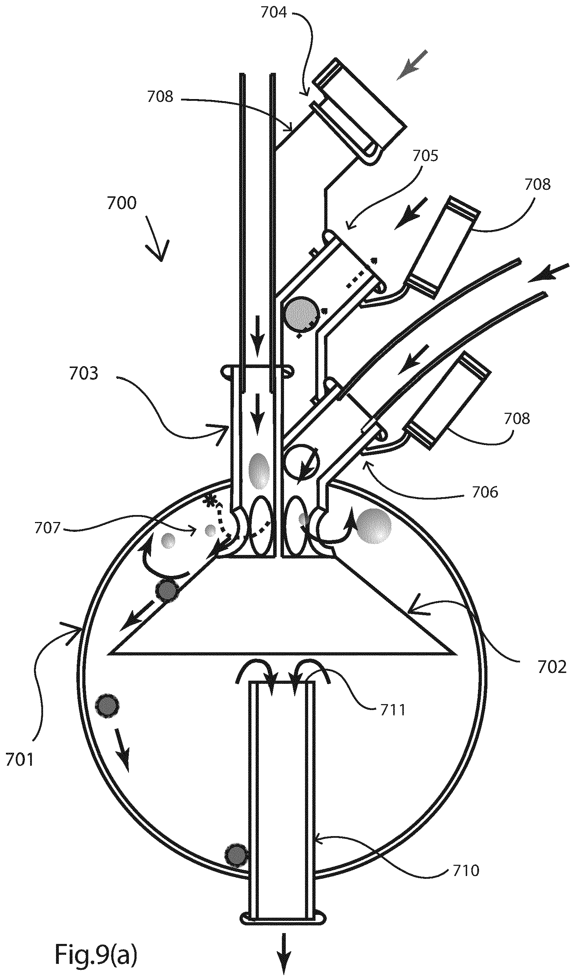

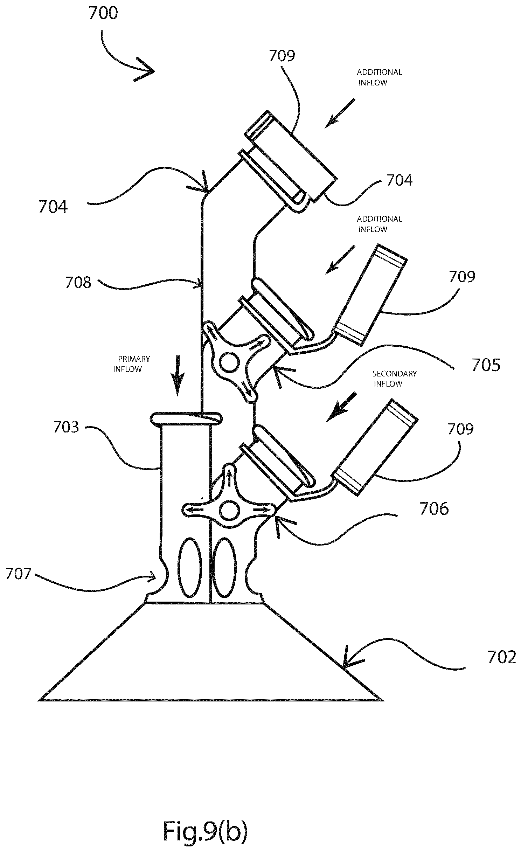

Referring to Figs. 9 (a) and (b) a trap 700 has a chamber 701 with a diverter 702 and an inlet 703 with a number of inlet ports in a staggered arrangement. In this case there are inlet ports 703, 704, 705, and 706, and in this case a single inlet conduit 708 has two ports (704 and 705). This arrangement allows excellent flexibility and in particular a multitude of fluid lines can be attached distal to the diverter 702, the benefit being that phase separation can occur in a position that subsequently enters the chamber by flowing through the diffuser 707 and over the diverter 702. The port 703 is used for primary inflow, the port 706 for secondary inflow, and the ports 704 and 705 for additional inflows. There is compactness and simplicity of manufacture where a single conduit has multiple ports feeding into it. It is preferable that the ports which are closest to the chamber, namely the ports 703 and 706, are used in a typical use, whereas the ports 704 and 705 which are further away and communicating with a single conduit 708 are used exceptionally. Any or all of the ports have a cap 709 for selective sealing.

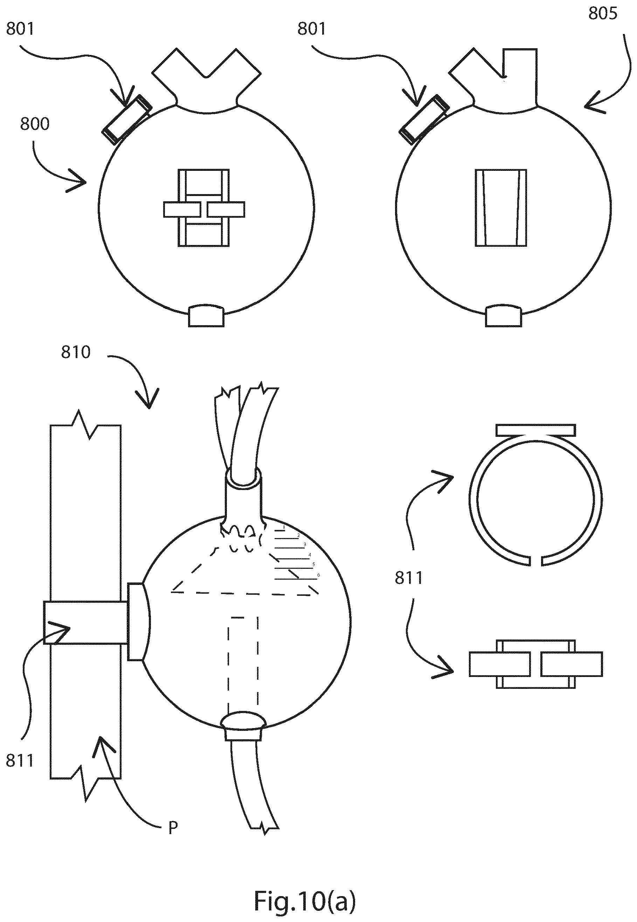

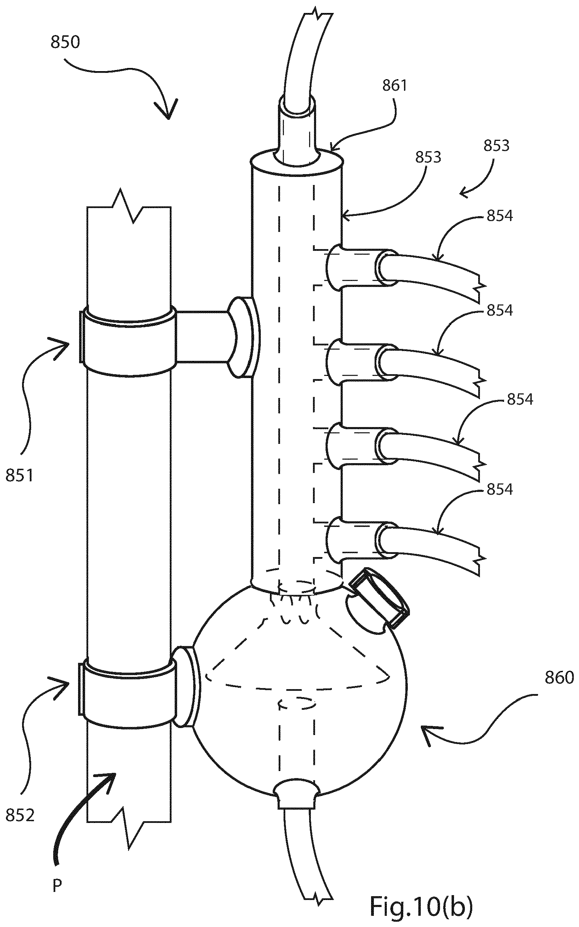

Figs. 10 (a) and (b) is a set of views of traps with arrangements to fix its position relative to an IV pole, or to be fixed. A trap 800 has a mounting fixture 801 arranged to encompass a pole, and a trap 805 also has such a bracket but a different inlet port arrangement. A trap 810 has a mounting fixture 811 half way between the inlet and outlet, shown mounted to a pole P. A manifold trap 850 has a pair of mounting fixtures 851 and 852 arranged to support to a pole both a chamber 860 and an elongated inlet 853 with an inlet conduit 861 and several inlet ports 854, extending radially from the conduit 853. The apparatus 850 is fixed to the chamber 860 in the lowermost position, such that fluids flow in through the manifold's plurality of inlet ports or inlet conduit 861, flowing downwards through the chamber 860.

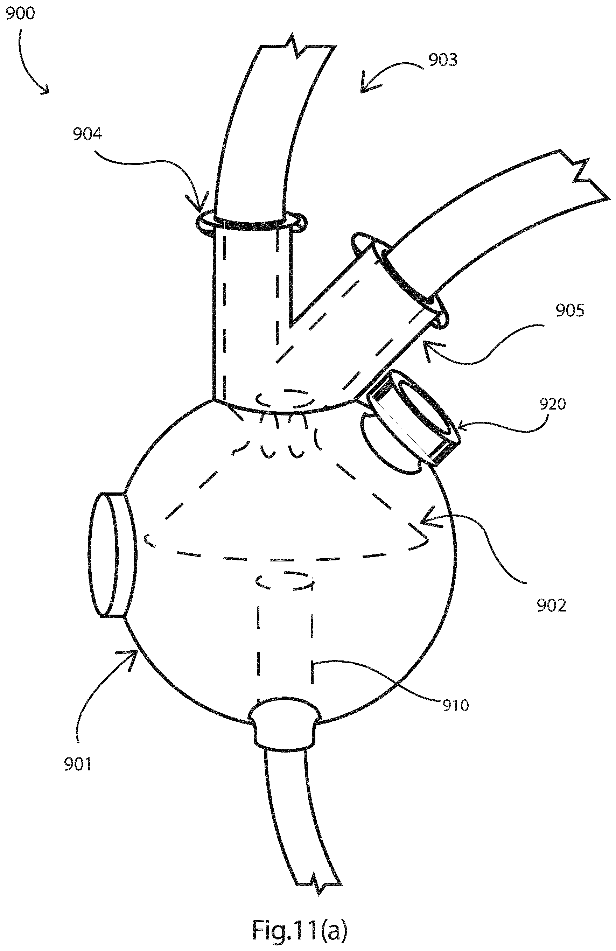

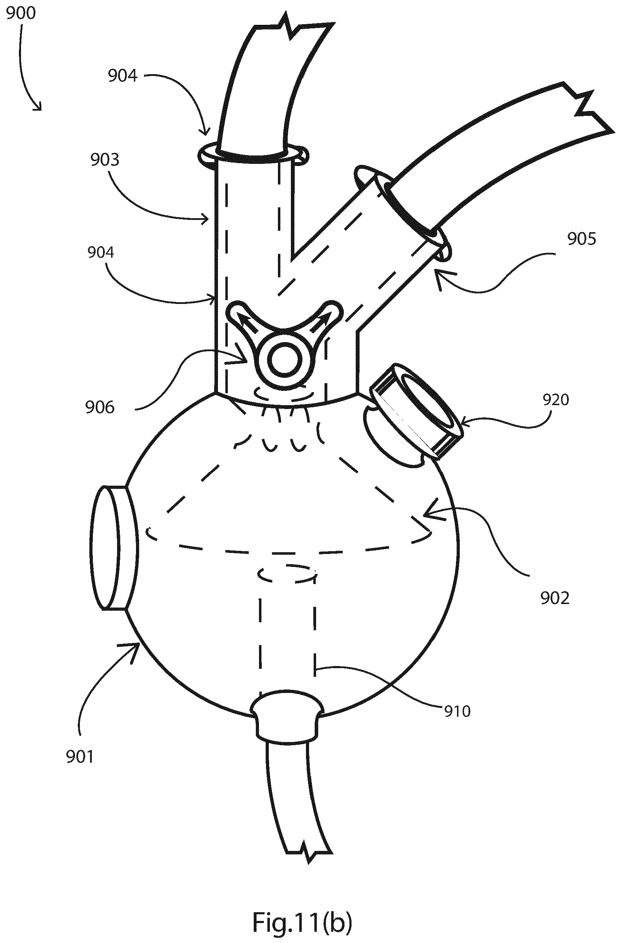

Referring to Figs. 11(a) and (b) a trap 900 has a chamber 901, a diverter 902, and an inlet 903 having inlet ports 904 and 905. In this case there includes a stopcock valve 906 to control flow between inlet ports 904 and 905, as well as flow into the chamber 901 for each port. It may fully or partly close flow from each port under manual control. The stopcock valve 906 is controlled by an external levered handle that rotates a barrel within a matching chamber along a central axis. The chamber 901 has a venting port 920. As for the above embodiments there is an outflow tube 910 with an inlet internally within the chamber 901.

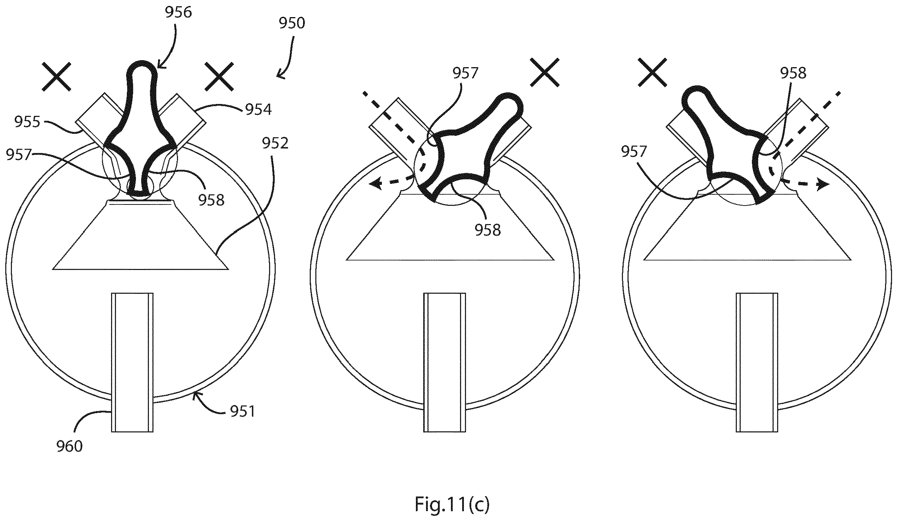

Referring to Fig. 11(c), a trap 950 has a chamber 951, a diverter 952, an outflow tube 960, and two inlet ports 954 and 955 with a valve 956 at their confluence. The valve 956 has a concave surface 957 and a concave surface 954. These surfaces provide flow routes when the valve is pivoted to the relevant position, the concave surface 957 allowing flow through the port 955 when facing upstream into that port, and the concave surface 958 allowing flow through the port 954 when it is facing upstream into that port.

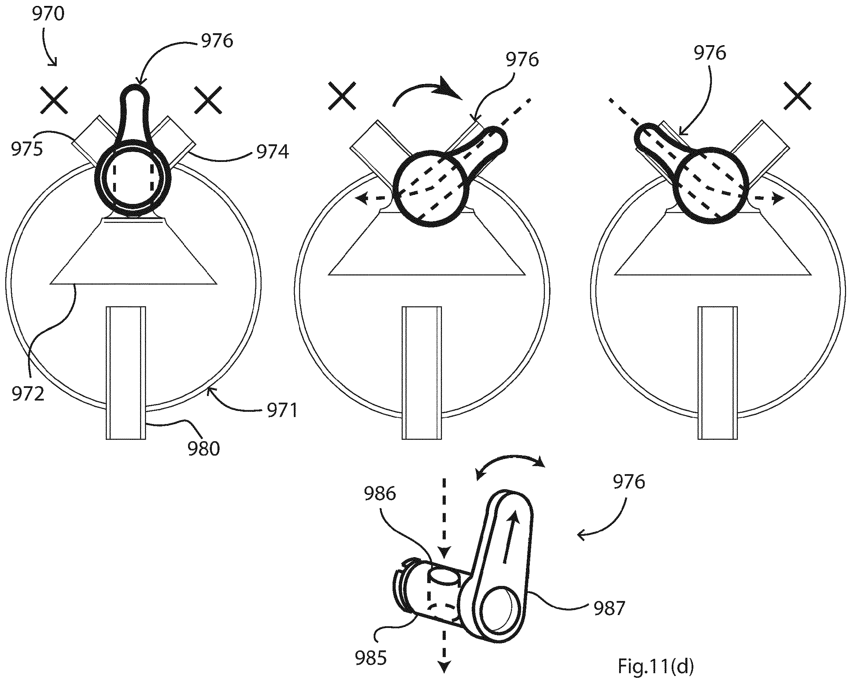

Fig. 11(d) shows a trap 970 with a chamber 971, a diverter 972, inlet ports 974 and 975, and a valve 976. The valve 976 has a barrel 985 with a transverse through hole 986 and a handle 987 to allow flow by pivoting to the positions where the hole 986 is aligned with one port or the other.

The barrel section is press-fit into place until a groove is located and fixed under friction pressures. When adequate force is applied in a rotational manner about the common central axis the stopcock barrel rotates, allowing a channel to selectively open or seal, depending on the position of the channel opening. When aligned to the direction of a fluid pathway the channel allows fluids to flow through the barrel of the stopcock. When not aligned to the fluid pathway the wall of the stopcock barrel forms a seal and does not allow fluids to flow through.

The stopcock apparatus valve may be formed as a single moulded plastic part that has a low coefficient of friction.

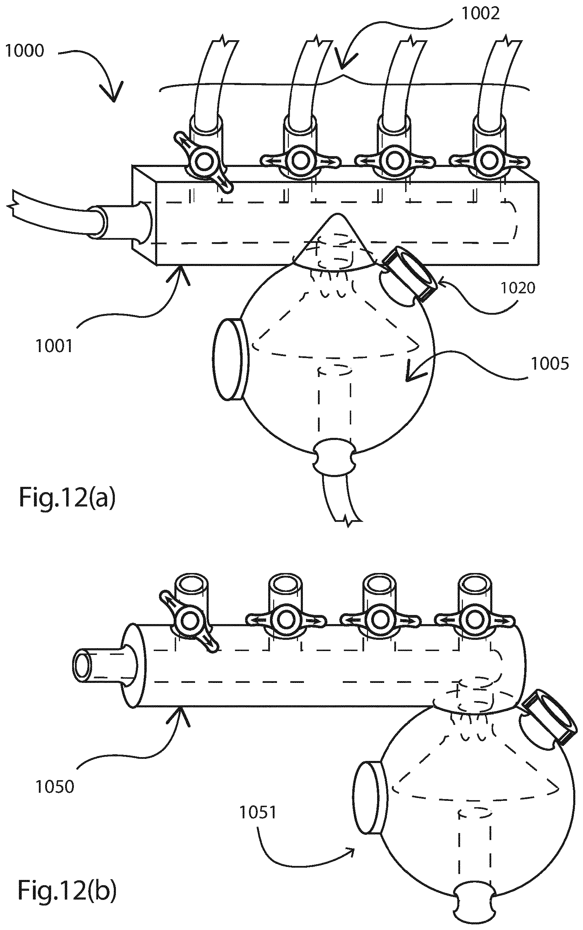

Figs. 12(a) shows a trap 1000 with a manifold 1001 with a plurality of individually-controlled valves 1002 controlling mixing and flow proportions into an inlet manifold 1001 *en route* to a chamber 1005. Fig. 12(b) shows an alternative arrangement in which a manifold 1050 is mounted at an end to a chamber 1051.

### Cap for Hazardous Drug / Toxic Biologics chamber

We describe below various vent ports which have a vent opening and a cap which is simple to put in place to seal the port, but either impossible or difficult to remove. This allows initial venting to purge a line, but sealing thereafter as a toxic fluid is administered. Where the cap is configured to be difficult to remove it may have a recess for rotation by a tool for removal, but cannot be removed by hand.

The feature of such a vent opening is applicable to a bubble trap with only a single inlet port, not necessarily one having an inlet with multiple ports. This aspect of the invention therefore applies to any bubble trap with a chamber for entrapment of bubbles.

Chemotherapy is a toxic drug that is commonly used to provide medical treatment for a broad range of cancers. It is most often delivered in intravenous format as a liquid drug that is infused directly into the vein of a patient, either in a hospital, clinic or home environment. Chemotherapy is a form of hazardous drug that is intended to destroy human cells in order to provide therapeutic solutions. Hazardous drugs can include a broad range of agents other than chemotherapy, including toxic biologics or drugs with carcinogenic, mutagenic and antineoplastic properties, all of which are harmful to human health.

It is preferable to administer toxic biologics or hazardous drugs (HD) in a closed system that does not allow exposure of environmental contaminants into the line nor exposure of the toxic substances to the surrounding environment. During intravenous (IV) infusion of the drug, trace amounts of the toxic fluid can escape the IV system and settle on surrounding surfaces. These can be in the form of liquid and/or gas and HD are observed to vaporize at room temperature and when exposed to ambient atmosphere. In modern healthcare, devices know as Closed System Transfer Devices (CSTD) are being utilised by healthcare workers to ensure HD are administered without exposure, to ensure the safety of both the patient and the healthcare worker. Adverse outcomes from exposure to HD in liquid or gas format can result in a range of health problems, including reproductive issues such as reduced fertility and increased risk of miscarriage, as well as dizziness, nausea, cancer and death. Repeated micro-dose exposure over long periods of time is shown to result directly in harm to the health of the healthcare workers in proximity to toxic biologics or hazardous drug infusions.

During administration it is not uncommon for the IV line to be opened to aspirate bubbles from the HD infusion lines as those bubbles pose a subsequent risk to the health of the patient. In removing air bubbles, or toxic vapour bubbles, the healthcare worker is placing their own health at risk. It is evident that devices to reduce exposure can have a positive impact on healthcare delivery.

Maintaining the closed chamber is important to protect healthcare workers and their patients from unknown or unintended contact with the chemotherapy or toxic agent that is flowing through the IV. In the setup of the IV, such as priming, where entrained air in the IV tube is expelled to ensure correct and safe performance of the procedure, it is necessary to have an open port to expel the air. Where a device is being used to purge problem bubbles from IV lines, in which the chamber is intended to retain the liquids and gases, a venting port is required to expel the air. The purpose of the open port is to allow air to escape the chamber so a required volume of fluids can enter the chamber. Once the chamber is filled with the required fluids it is then necessary to seal the chamber so the drug is not exposed in an unsafe environment such as at the bedside. Where blood or hazardous drugs are being administered, it is often preferred and safer to ensure that the chamber cannot be easily opened, to ensure no risk of exposing the toxic drug.

As such, a method to ensure a reliable seal is in place would potentially reduce opening of the chamber. Preferably the cap cannot be opened or removed by hand. Such a device, when fixed into position, would provide an airtight seal to ensure that no liquids or vapours would be able to transmit in or out of the chamber.

The cap can be push fit, rotation or latch mechanism, or any other mechanism, such that it can be placed into an active position and not removed easily. The cap may be fixed in place or it may be selectively sealable such that it can be opened to enable critical functions to be performed. It may seal off passage of fluids, vapours of environmental contaminants by press fit contact between mating surfaces, or it may utilise a sealing ring or membrane device.

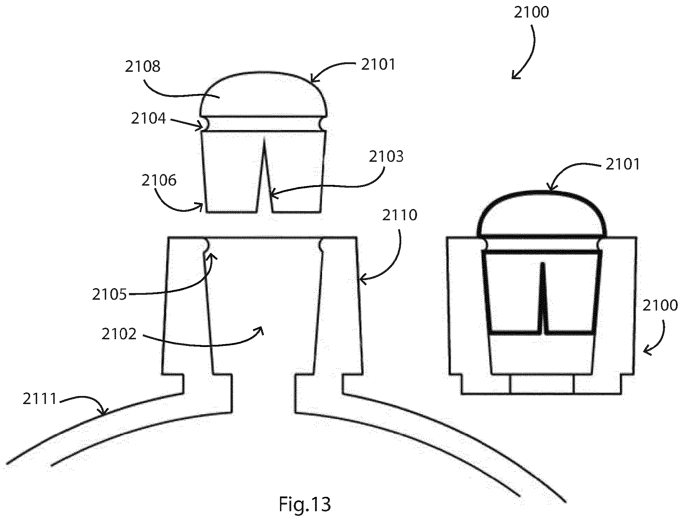

Referring to Fig. 13 a vent port 2100 comprises a cap 2101 which fits into a vent opening 2110, the cap 2101 being intended to seal the chamber opening 2110 in a manner which is difficult to open. The seal 2100 can form part or whole of a trap chamber that contains fluids that are intended to be separated from internal and external environments. By placing the cap 2101 into position, the opening 2110 is fully sealed with an air-tight seal and no fluids can pass through. The cap 2101 can be pushed into place, twisted, squeezed or in any other manner forcibly placed into the orifice opening.

In this embodiment the cap 2101 comprises downwardly-depending legs 2106 separated by a V-shaped gap 2103 to provide a split feature that is intended to flexibly deform when pushed into the mating vent opening 2110 of the chamber. The mouth of the opening 2110 has a radially inwardly-directed rim or protrusion 2105 that locks into position with a circumferential groove 2104 in the cap 2101. When fully in place, the downwardly and inwardly angled walls of the legs 2106 of the cap base are in contact with the internal surfaces of the walls of the chamber opening 2110 to form an airtight seal. The top of the cap 2101 has a convex surface 2108 that is intentionally difficult to grasp and thereby reducing potential for removal of the cap by human hand, such that the cap 2101 and the chamber 2111 are forcibly sealed and held together to form a sealed unit.

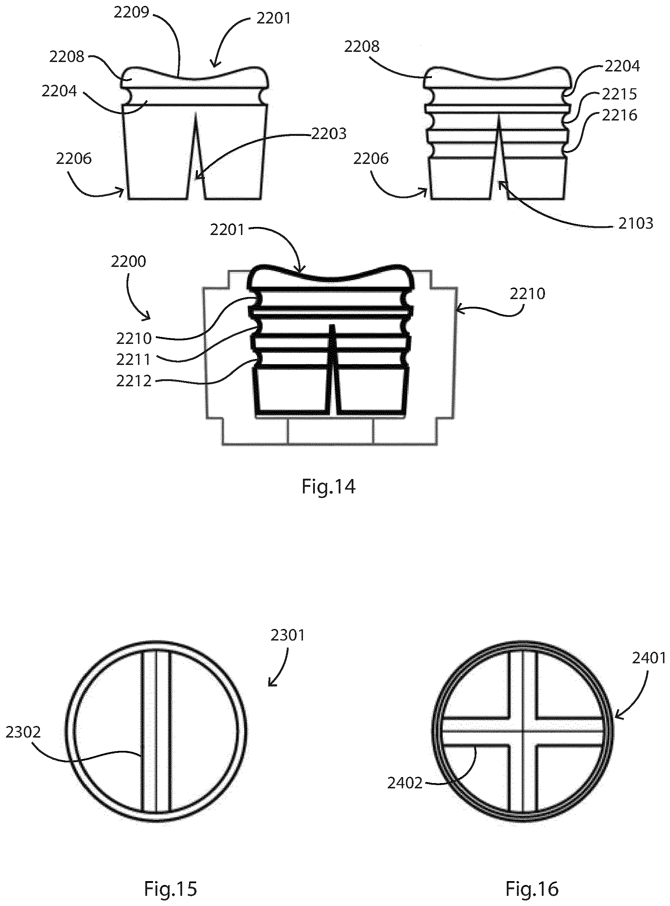

Fig. 14 is a set of views of an alternative vent 2200, having a cap 2201 and a vent opening 2210. In this case the cap 2201 has legs 2206 which have outer surfaces tapered inwardly to narrow on the end which is inserted into the opening 2100, and separated by a V-shaped gap 2203. Again, there is a circumferential groove 2204. In this case, however, the top 2208 has a lower profile, with a shallow depression 2209 diametrically across its centre. Also, there are additional circumferential grooves 2215 and 2216 below the groove 2204. The opening 2210 has corresponding ridges 2210, 2211, and 2212 to engage tightly with the grooves 2204, 2215, and 2216.

In other examples there may be a pair of cross-shaped cut-outs akin to the cut-out 2203, allowing more uniform flexibility around the circumference.

As shown in Figs 15 and 16 a cap 2301 may have a diametrically-extending slot 2302 for gripping by a tool, or a cap 2401 may have a cross-shaped groove 2402 for engaging a tool.

The cap, preferably with a split base, may have any of a variety of shapes and materials. Some materials may be selected for their low or high friction coefficient properties, or elasticity properties, such that the material can distort or flex as required to fit into the opening. The split may be of any of various notched shapes to enable flexible distortion. The material may be required to return to its given geometry once a force is removed.

The cap top may be a material that is the same or different to the cap base material. The cap top shape may be round, flat, convex, concave or any other geometric shape that is intentionally difficult to grip or pull.

As noted above the cap may have one or more notches that interface with protrusions, such that, when the notch moves past the top edge of the protrusion, the cap is rigidly held and locked into position using friction or surface to surface contact.

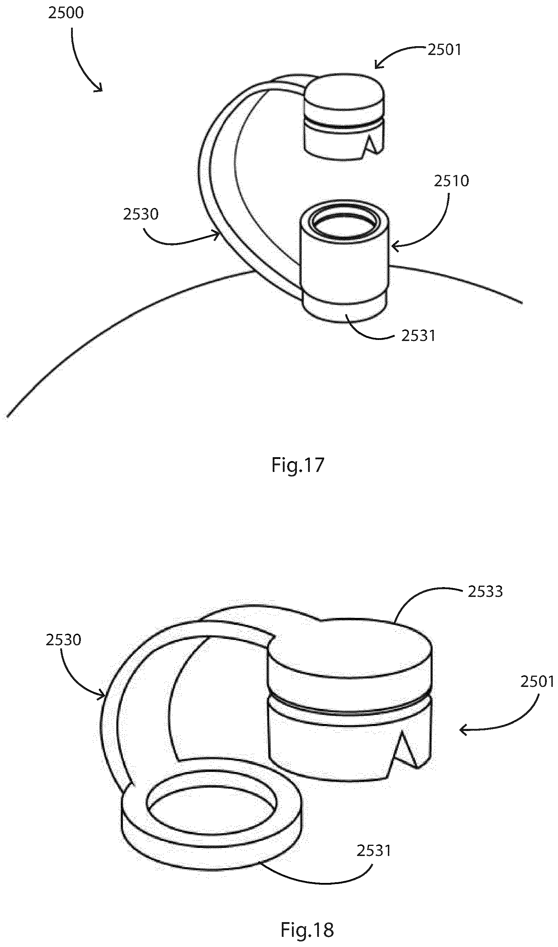

For convenience it is practical to have the cap located on or near the device that requires closure. In one embodiment shown in Figs. 17 and 18 a cap 2501 is attached to the opening 2510 of a chamber 2511 by means of a flexible connector or ribbon 2530, such that the cap can be manoeuvred into the opening. The ribbon can be attached to the chamber by bonding, connecting, clipping or moulded in place. The cap 2501 is in this case attached by the ribbon 2530 to the opening by means of a ring 2531 that slips over the opening 2510. The purpose of the ring is to enable the cap to be twisted into place or pushed into place, with the ring rotating to a convenient position and without shearing or twisting the ribbon 2530.

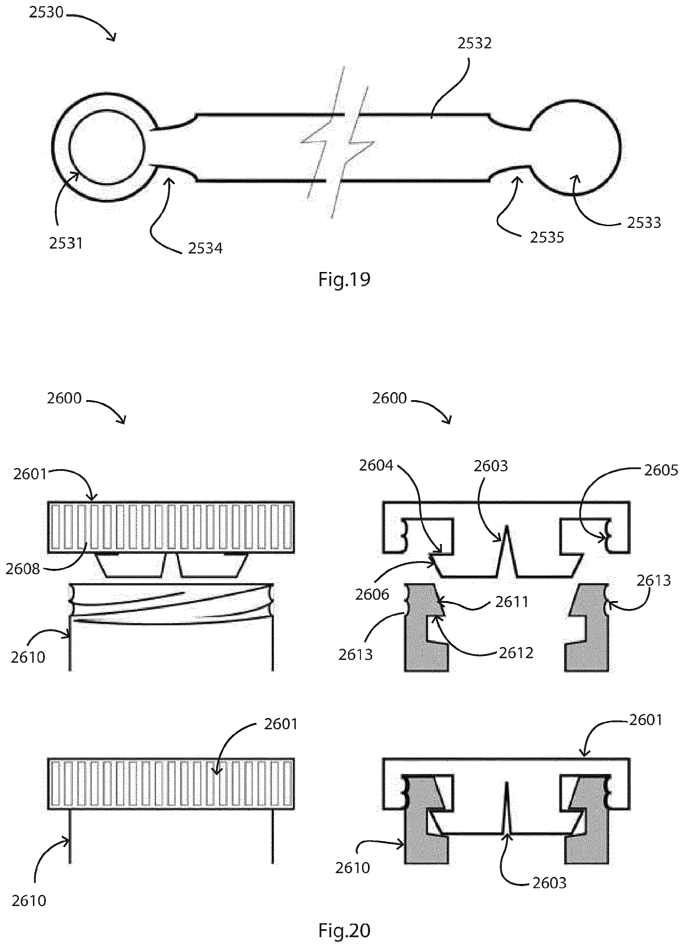

In one embodiment shown in Fig. 19 the ribbon 2530 comprises the ring 2531 at the inner end for engaging the opening and a disc 2533 at the outer end for engaging the cap. These ends are connected by a band 2532 with a narrowing geometry or necks 2534 and 2535 at the inner and outer ends respectively. These form a weak link so that it can easily pulled, either by hand or an appropriate tool, and disconnected once the cap is in its permanent location. The reason for removing the ribbon is to make it less likely that a person can pull on the cap and remove it from its intended permanent location as a seal.

The cap can be placed into its permanent home location to seal the chamber during the priming process and the ribbon section can be snapped off to indicate the chamber is sealed. In one embodiment the material is flexible, in another embodiment the material is rigid, in another embodiment the material is a bright colour to identify its location.

In a further embodiment the cap is formed with an internal thread that is of the common Luer type, such that it can be rotated and sealed in place on the outside section of a Luer fitting, which is common practice in medical procedure.

In a further embodiment the cap is formed with a push-fit that uses surface-to-surface friction contact to be sealed in place on the outside section of a fitting.

In another embodiment shown in Fig. 20 in a port 2600 a sealing cap 2601 connects to the port opening 2610 by a Luer thread 2605 that mates with a thread 2613 on the outer face of the port, namely on the external surface of the opening 2610. The cap 2601 has a split 2603 on an internal section, the purpose of which is to distort and narrow when the radially outwardly-facing cap face 2606 is in contact with the port opening edge 2611. When fully rotated into place the internal cap face 2604 is in contact with the port face 2612, and as the flexible port expands the cap 2601 is trapped on the port opening 2610. By rotating the cap 2601 on the Luer threads it still remains fixed in its permanent location due to the internal split section being trapped by the contacting faces 2604 and 2612, essentially not removable once in place. The cap 2601 has hand-grip knurls 2608 on the outer face to support tightening of the cap device by hand initially.

In another embodiment the cap is attachable by bonding, connecting, moulding or some other process such that it is always connected to the device with the open port.

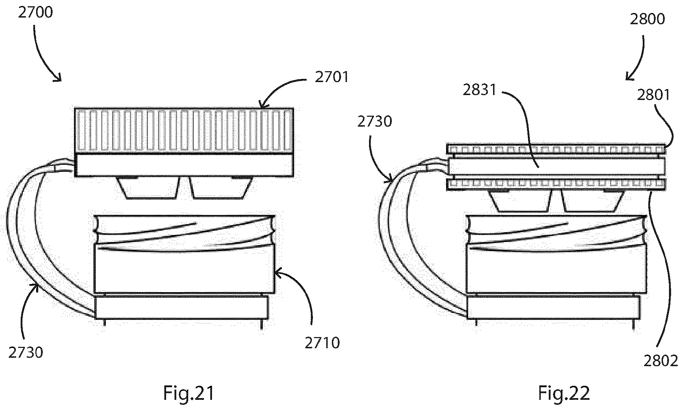

In another embodiment shown in Fig. 21 a cap 2700 has many features equivalent to those of Fig. 20, but in this case the cap 2701 is linked to the opening 2710 by a ribbon 2730. Fig. 22 shows a variation, in which a cap 2800 has a cap 2801 which is similar to the cap 2701 except that it has a pair of narrow knurled grip surfaces 2801 and 2802 separated by a ring 2831 which is integral with the ribbon 2830.

### Uses

The following describes uses of any of the apparatus' described herein. Fluids flow into the chamber through the primary inflow port and are diffused into the chamber through a plurality of diffuser holes. The diffuser holes are located in proximity to where the inflow tube abuts the conical diverter, distal to the conical diverter such that fluids enter the chamber via the inflow tube, through the diffuser holes and over the conical diverter. These one or more diffuser holes may be circular, oblongs or slots that are arranged on the body of the device in such a manner that the diametrical sum of the plurality of holes is equal to the inflow tube diameter so as not to impact flow rates into the chamber. Where required, the primary fluids can also be combined with fluids from a secondary inflow port before being diffused into the chamber, which may encourage mixing and aspiration of the two fluids. The fluids may flow in under force of gravity or may be induced by a pump or any other means of inducing flow rate when the device forms part of an IV administration system.

A major advantage of allowing primary and secondary flows to enter and mix in the chamber is that any naturally-occurring degassing is prohibited from entering the tubing distal to the device. The apparatus can act as an inline air aspirator that promotes efficient release of the entrained gas from the liquid phase. Due to controlled release of gas from various fluids within a closed chamber there is lower risk of the gases passing into the outflow port. An advantage of this would be a significant reduction in air-in-line alarms of the electronic IV pump device that monitors fluids for problem bubbles, which can result in interruptions to medication delivery.

Another advantage is less requirement for attending nursing personnel to break into the normally closed system, with less time spent to manipulate the gas into a convenient position for removal and no requirement for additional equipment to aspirate the captured gas leading to efficient and lower cost benefits for healthcare providers. Another advantage is that the harmful drug vapours are retained within the sealed chamber of the device to protect healthcare workers from exposure to toxic drugs.

Having a plurality of infusion ports on one closed body device allows a mixture of solutions to be administered without presence of problem gas bubbles in the mixture.

Diffusing the solution into the chamber promotes buoyancy forces to overcome the flow rate of the fluids, such that any gas bubbles are forced to rise to the top of the fluids more readily. By diffusing the bubbles and allowing microbubbles to coalesce on entry, the effects of buoyancy are increased. An advantage is that the gas bubbles are less likely to travel into the body of fluids in the chamber, instead tending to float to the air-liquid interface region where the bubble collapses.

A further advantage of the device is utilising the conical diverter to redirect particles of fluid towards the outer edges of the fluid body upon entry to the chamber. At the outer edges of the chamber, any entrained air is furthest from the intake end of the outflow port and laminar flow is induced as particle movement slows such that bubbles are more likely to rise under buoyancy effects. Also, any entrained particles are more likely to drop to the lower portions of the chamber, furthest from the outflow port.

The primary inflow is used to flood the chamber with the primary fluid. In this case the outflow port may be closed by means of a tap or clamp to stop flow out from that port and the secondary inflow tube is opened to allow a pressure differential such that the primary fluid is encouraged to flow into the chamber and all entrained air is expelled out of the open secondary inflow tube. The secondary inflow tube can then be sealed, for example with a cap, seal or activation of a tap. With the secondary tube closed, the outflow tube can then be opened to create a liquid pathway from primary inflow tube to outflow tube. Further, a secondary inflow tube can then be attached to the secondary inflow port such that a mixture of the primary and secondary fluids can be infused into the chamber and the combined solution can be expelled through the outflow tube.

In another embodiment the venting port or secondary inflow tube can feature a normally sealed or self-healing membrane. Such a membrane may be formed from rubber, silicone or other material that can elastically deform and return to normal shape. This membrane, which is normally closed but can deform to allow passage of fluids through a channel, may be acted upon by a needleless connector that forces the normally sealed membrane to deform and open its channel such that liquids can pass through. In such a case the liquids can be injected into the chamber by a needleless syringe, Closed System Transfer Device or another IV tubing with suitable connector may be attached by means of a Luer connection or push fit or other connection.

An advantage of having a normally sealed membrane at the secondary inflow port is that the toxic liquids that may be contained in the secondary connection device can pass into the chamber via the secondary inflow port while maintaining a sealed system.

In one embodiment the primary and secondary inflow tubes are separated by a dividing wall, to encourage laminar flow profiles such that each fluid enters the chamber in its own channel without mixing. These separate fluids are diffused into the chamber through a plurality of entrance holes in the diffuser section. An advantage of this embodiment is that the primary inflow can have an IV line attached and be used to fill the chamber with fluids while the independent secondary inflow tube can remain open, acting as a venting port for the entrained air until the chamber is fully primed. In use, the secondary can then be selectively sealed or it can have an IV line of fluids attached to be used as a secondary inflow, whereby the fluids flow into the chamber through the diffuser and diverter as previously described.

In a further use, the primary and secondary fluids, or additional fluids, are mixed together before the diffuser. This mixing is to encourage degassing between the liquids.

In another use the separation of the liquid and gas phase of the fluids, as well as particles and artefacts, may be accelerated by inclusion of a vortex screw that directs the fluid flow to the outside walls of the fluid channel in a swirling motion that encourages the air and particles towards the outer regions of the fluid body. In this case the outer region of the fluid body is the most central region of the chamber. When the two or more fluids combine in this way the particles crash together and create a turbulent mixing regime where air bubbles of various sizes are formed. These bubbles along with the combined solution enters the chamber through the diffuser holes and are carried through the fluid body as intended, with gas bubbles floating upwards under buoyancy forces and particles sinking due to relative weight in the fluid body.

In a further use, the outflow port may be protected from artefact ingress by incorporating a filtration membrane that may be fixed to the surface of the diverter, the top of the outflow tube or any other internal surface of the chamber. The advantage of using a filter membrane on the diverter surface is an increased surface area that is less prone to blockages, the membrane can swell as intended without overly impacting the intended flow rate while solid particles are prevented from entering the outflow tube that leads to the pump or patient.

In one use a primary inflow tube is connected to the air aspirator device by means of a Luer lock. In another embodiment the primary inflow tube is connected to the air aspirator device by means of a push-fit. In another embodiment the primary inflow tube is connected to the air aspirator device by means of bonding such that the primary tube is permanently fixed in place on the tubing.

In one use the secondary and additional inflow tube is connected to the air aspirator device by means of a Luer lock. In another embodiment the secondary and additional inflow tube is connected to the air aspirator device by means of a push-fit. In another embodiment the secondary and additional inflow tube is connected to the air aspirator device by means of bonding such that the secondary and additional tube is permanently fixed in place on the tubing.

In another use the primary inflow port and secondary inflow port are used solely for infusing fluids into the chamber through the diffusion holes of the air aspirator device. In this case there may be a separate venting port, the separate venting port having a hole feature that allows the air entrained at priming to be vented out of the chamber through this hole feature, up the channel and out through the venting port only. In this embodiment the primary and secondary ports only allow the inflow of the infusion fluids. The primary and secondary ports are sealed with a cap, tap or other method, or may be filled with fluids while the venting port is open, thereby creating a pressure gradient that forces air to escape through the open port.

In another use this venting port may include a self-healing normally closed membrane as previously described. The advantage of this embodiment is that gases that are aspirated may collect in the upper section of the chamber. Where an excess of gas is collected there may be a requirement to vent these gases without stopping the infusion process. In this situation a suitable Closed System Transfer Device (CSTD) may be attached to the venting port that actuates the membrane to allow passage of gases from the chamber into the CSTD and the entrained gas are syphoned away into a device e.g. a CSTD, syringe or other device.

The advantage of having a series of plurality of inflow tubes is it allows more than one fluid to be infused through the air aspirator device at one time. This can increase efficiency of medication delivery as well as provide a single point of observation for clinicians to monitor the infusion lines for air or other impediments that affect the overall delivery of IV fluids. Another advantage of this embodiment is to reduce manufacturing steps and costs so as to provide a cost-effective solution for healthcare providers.

In many uses, the primary line is back-primed into the secondary line to purge the secondary line of initially entrained air. Typically the secondary line is connected to the primary line by means of a y-connector. The secondary fluid bag is held in a position lower than the primary fluid bag, which forces liquid into the secondary line. Using this method, primary liquid is allowed to fill the entire secondary line. Once the secondary line is completely cleared of air, the secondary bag of fluids is lifted higher than the primary bag, which forces the liquid in the secondary line to be dominant and it flows into the primary line. In some cases a mechanical IV pump is used to control the flow rates of the primary and secondary fluids. Thus, the primary fluid and the secondary fluid will flow as a mixture into the chamber of the invention and air aspiration of the mixture will take place as described.

In one use the primary fluids enter the chamber with the exit port tube clamped, which stops flow in that direction; and the secondary port is opened, which promotes flow in that direction. As the chamber is primed with fluids from the primary line, the primary fluids fill the chamber, and travel through the diffuser holes and into the secondary line where the secondary line priming is carried out as described. The advantage of this is that it is normal clinical practice to back-prime a secondary line in this manner to expel entrained air. A second advantage is that the entrained air is expelled up from the chamber, through the secondary line and into the secondary IV bag where it is trapped, effectively maintain the closed system that is integral to patient and staff safety. Once the chamber is primed and the secondary line is primed the flow is reversed so the secondary can flow into the chamber as required to complete the treatment. The apparatus can then remove air bubbles from the primary and secondary lines as described above.

In some uses a two-way or three-way stopcock tap device is selectively opened or closed to create a passage between the primary port, the secondary port or the chamber. An advantage of including a two-way or three-way tap device is that the flow of fluids from the primary line into the secondary line can be controlled and arrested conveniently. Another advantage is that, in the event of an adverse reaction of the patient to the drugs in the secondary line, that port can be quickly and conveniently closed to stop further infusion of that drug. Another advantage of selective opening and closing of the ports is that it can allow priming of the secondary line to take place before the fluids enter the chamber. The combined fluids can then enter the chamber through the diffuser holes and the air is aspirated as described previously.

### Alternative Embodiments

In one embodiment this air aspirator device is standalone and disposable. In another embodiment this air aspirator device is a component of a primary giving set and disposable along with the giving set. In a further embodiment the air aspirator forms a solid shape. In a further embodiment the air aspirator is formed from sub-parts that are manufactured by means of injection moulding process. In a further embodiment the air aspirator sub-parts are assembled into a solid component.

In a further embodiment the air aspirator is assembled onto the chamber section, the purpose of which is to capture the gases that are aspirated. The air aspirator is located at the upper section of the chamber such that all fluids enter the chamber from the top and exit the chamber through the outflow port at the bottom end. The apparatus is intended to function in any orientation and as such the terms 'top' and 'bottom' relate to the inflow and outflow respectively, not the absolute coordinates in a fixed sense.

In a further embodiment the outflow port is elongated with the intake end of the outflow port located largely centrally of the chamber. One advantage of having an elongated outflow port with an intake end located in the centre of the chamber is that the particles that have collected in the fluids may be positioned at the lowest position of the chamber where it abuts the outflow tube and those particles may be restricted from entering the outflow tube. Other particles may be free floating in the liquid body due to buoyancy.

In another embodiment a hydrophilic membrane abuts the intake end of the outflow tube to prevent ingress of floating particles or solid artefacts. In another embodiment a hydrophilic membrane is attached to the base surface of the diverter body. This membrane is shaped to surround the intake end of the outflow tube to prevent ingress of floating particles or solid artefacts. In a further embodiment the surface of the diverter is polished to discourage 'sticking' or propagation of bubbles on the diverter surface.

In another embodiment the trap comprises a chamber with an attachment device that fixedly attaches the device to a local surface. In practice, where a primary line is attached to the trap, the inflow ports of the air aspirator component is positioned in the uppermost position, while the outflow port is positioned in the lowest position. Thus the device may be fixed in that position by means of a split ring, clip or other fixation method. The advantage of fixing the position of the device is that additional infusion tubes can be attached easily and the device can be located at a convenient height for clinical staff.

In another embodiment the chamber of the trap is spherical. In a further embodiment, the chamber is square. In a further embodiment, the chamber is oblong. In a further embodiment, the chamber is triangular.

An advantage of the device being spherical is that it enables the trapped gas to collect away from the elongated exit tube in any orientation. An advantage of the spherical chamber is that it minimises the priming volume of the device which can be important where valuable fluid resources such as drugs or blood products are being infused. An advantage of the fixed position device being oblong or taller is that the chamber can retain a greater volume of gas.

In a preferred embodiment the trap is free to move and rotate in any orientation and is not fixed in any particular position. In a further embodiment, the chamber is opaque. In a further embodiment, the chamber is translucent. In a further embodiment, the chamber is transparent.

One advantage of the chamber being transparent is that fluids and gas formation can be easily observed and monitored. In another embodiment the apparatus comprises a measurement system. In use the measurement system takes a reading of the amount or level of trapped air or collected gas bubbles in the chamber. In a preferred embodiment the measuring system is a gradient rule.

One advantage of a measurement system is that the volume of gas present in the chamber of the apparatus may be accurately measured. In another embodiment the plurality of inflow ports can be turned on or off depending on whether an infusion tube is connected for the purpose of transferring fluids into the chamber. In this case each port would be capable of being opened or closed independently of each other by means of a tap mechanism.

The advantage of having a two-way or three-way tap is that each port can be shut off from the adjoining port and fluid losses can be minimised as a result. Where a line of infusion fluids is attached, the tap between the proximal port to the infusion port would be closed, thus ensuring all fluids only enter the intended infusion port. When an additional infusion line is added to the proximal port the tap can then be opened to enable mixing of the fluids. The fluids would be free to flow from the uppermost port down through the plural open ports and into the chamber located at the lowermost position.

One advantage of placing the chamber at the lowermost position is that all fluids would flow down into the chamber and the maximum efficiency in releasing the entrained gases would be possible. The degassing of the plural fluid solution would occur in the ports and in the chamber through the diffuser device. The gases would be trapped in the uppermost section of the chamber and the fluid body would remain largely free of problem bubbles.

In another embodiment the apparatus is formed of a malleable material. In another embodiment the apparatus is formed of rigid material. In a preferred embodiment the rigid material is a polymer. In another embodiment the apparatus is formed of individual component parts. Component parts would require construction to form the complete apparatus prior to use. In another embodiment the apparatus is capable of being manufactured as a single unit.

In another embodiment the apparatus is manufactured by means of 3D-printing. In another embodiment the apparatus is manufactured by means of injection moulding. In a preferred embodiment the apparatus can connect with standard connection methods with multiple types of tubing including a variety of pumps. In another embodiment the device is scalable in size, the optimal size being assessed by the user dependent on the use case. In a preferred embodiment the device can capture and retain 1cc or more of gas automatically and retain the gas in a sealed chamber. This is considered beneficial where hazardous drugs, or toxic biologics such as blood products, are used so the vapours are not released to atmosphere.

The invention is not limited to the embodiments described but may be varied in construction and detail within the scope of the claims.

## Claims

1. A gas trap apparatus for medical fluids, the apparatus comprising a bubble entrapment chamber (101), an inlet (103) to the bubble entrapment chamber with a plurality of inlet ports (104, 105), and an outlet with at least one outlet port (110),
wherein the inlet ports (104, 105) are adapted to allow fluid flowing through the inlet ports to mix as they enter the chamber (101),
wherein the apparatus comprises a flow diverter (102) in the chamber arranged to divert flow from the inlet (103),
**characterized in that**,
the flow diverter (102) is arranged to divert flow from the inlet (103) to have a lateral directional component with respect to a longitudinal flow direction from the inlet and away from the outlet port and towards an internal surface of the chamber, and in which the diverter has a shape arranged to divert flow distally from the inlet and radially before the flow is past the diverter and can move radially inwardly towards the outlet.

2. A gas trap apparatus as claimed in claim 1, wherein the inlet includes a turbulence-inducing flow barrier (120), and optionally at least one inlet port includes a turbulence-inducing flow barrier (120).

3. A gas trap apparatus as claimed in claim 1 or claim 2, wherein the chamber includes a hydrophilic membrane (603).

4. A gas trap apparatus of claim 3, wherein the membrane (603) is mounted at a distal end of the diverter.

5. A gas trap apparatus as claimed in any preceding claim, wherein at least one port includes a valve (705) for fully or partly closing the port.

6. A gas trap apparatus as claimed in claim 5, wherein a plurality of inlet ports comprises a valve, and the apparatus comprises a mechanism for interconnection of the valves and their synchronized operation.

7. A gas trap apparatus as claimed in claims 5 or 6, wherein the inlet comprises a single valve (906) which is mounted to alternatively open and close a plurality of the inlet ports.

8. A gas trap apparatus as claimed in any preceding claim, wherein the inlet comprises a common conduit (708, 853) for a plurality of ports (709 -706, 854), and optionally said common conduit extends parallel to the longitudinal direction.

9. A gas trap apparatus as claimed in any preceding claim, wherein the apparatus comprises a venting port (920) which is sealable, the venting port comprising a vent opening (2110) and a cap (2101) which is engageable with the vent opening.

10. A gas trap apparatus as claimed in claim 9, wherein the cap and the vent opening have inter-engaging features (2104, 2105) which are engageable by pushing the cap into the opening.

11. A gas trap apparatus as claimed in claim 10, wherein the cap has a curved exposed surface without a hand grip.

12. A gas trap apparatus as claimed in either of claims 10 or 11, wherein the cap is linked with the vent opening by a ribbon (2530), wherein the ribbon has a ring engaged around the vent opening to allow rotation about an axis of the vent opening, and wherein the cap is configured for closure only without removal, and optionally the cap and the vent opening have inter-engaging features (2606, 2612) which provide a snap-fit lock with pushing of the cap into the vent opening.

13. A gas trap apparatus as claimed in any preceding claim, wherein the inflow tube comprises a diffuser upstream of the diverter, said diffuser comprising a ring of apertures around the inflow tube circumference.

14. A gas trap apparatus as claimed in claim 13, wherein the inlet ports merge at a confluence space and the chamber comprises said diffuser comprising apertures (107) around the inflow tube for inflow of fluids into the chamber with a lateral dimensional component, the inlet ports (104, 105) have a diameter in the range of 1.5 mm to 8.0 mm, and the distance between an upper end of a confluence space between the ports (104, 105) and the diffuser apertures (107) is no more than 20mm.

15. An infusion apparatus comprising a gas trap apparatus of any preceding claim and infusion lines linked with the inlet ports and an infusion line linked with the outlet.

## Patentansprüche

1. Gasfallenvorrichtung für medizinische Fluide, wobei die Vorrichtung eine Blaseneinschlusskammer (101), einen Einlass (103) in die Blaseneinschlusskammer mit einer Vielzahl von Einlassöffnungen (104, 105) und einen Auslass mit mindestens einer Auslassöffnung (110) umfasst,
wobei die Einlassöffnungen (104, 105) dazu angepasst sind, zuzulassen, dass sich durch die Einlassöffnungen fließendes Fluid vermischt, wenn es in die Kammer (101) eintritt,
wobei die Vorrichtung einen Strömungsumleiter (102) in der Kammer umfasst, der dazu angeordnet ist, eine Strömung von dem Einlass (103) her umzuleiten,
**dadurch gekennzeichnet, dass**
der Strömungsumleiter (102) dazu angeordnet ist, eine Strömung von dem Einlass (103) her umzuleiten, sodass sie eine Querrichtungskomponente mit Bezug auf eine Längsströmungsrichtung von dem Einlass her und von der Auslassöffnung weg und zu einer Innenoberfläche der Kammer hin aufweist, und wobei der Umleiter eine Form aufweist, die dazu angeordnet ist, eine Strömung distal von dem Einlass her und radial umzuleiten, bevor die Strömung den Umleiter passiert und sich radial nach innen zu dem Auslass hin bewegen kann.

2. Gasfallenvorrichtung nach Anspruch 1, wobei der Einlass eine turbulenzauslösende Strömungsbarriere (120) enthält und optional mindestens eine Einlassöffnung eine turbulenzauslösende Strömungsbarriere (120) enthält.

3. Gasfallenvorrichtung nach Anspruch 1 oder Anspruch 2, wobei die Kammer eine hydrophile Membran (603) enthält.

4. Gasfallenvorrichtung nach Anspruch 3, wobei die Membran (603) an einem distalen Ende des Umleiters angebracht ist.

5. Gasfallenvorrichtung nach einem der vorhergehenden Ansprüche, wobei mindestens eine Öffnung ein Ventil (705) zum vollständigen oder teilweisen Verschließen der Öffnung enthält.

6. Gasfallenvorrichtung nach Anspruch 5, wobei eine Vielzahl von Einlassöffnungen ein Ventil umfasst und die Vorrichtung einen Mechanismus zur Zusammenschaltung der Ventile und zu ihrem synchronisierten Betrieb umfasst.

7. Gasfallenvorrichtung nach Anspruch 5 oder 6, wobei der Einlass ein Einzelventil (906) umfasst, das so angebracht ist, dass es eine Vielzahl der Einlassöffnungen abwechselnd öffnet und verschließt.

8. Gasfallenvorrichtung nach einem der vorhergehenden Ansprüche, wobei der Einlass eine gemeinsame Leitung (708, 853) für eine Vielzahl von Öffnungen (709 -706, 854) umfasst und optional die gemeinsame Leitung sich parallel zu der Längsrichtung erstreckt.

9. Gasfallenvorrichtung nach einem der vorhergehenden Ansprüche, wobei die Vorrichtung eine Abzugsöffnung (920) umfasst, die abdichtbar ist, wobei die Abzugsöffnung einen Lüftungsdurchlass (2110) und eine Kappe (2101) umfasst, die mit dem Lüftungsdurchlass in Eingriff bringbar ist.

10. Gasfallenvorrichtung nach Anspruch 9, wobei die Kappe und der Lüftungsdurchlass ineinander eingreifende Merkmale (2104, 2105) aufweisen, die in Eingriff bringbar sind, indem die Kappe in den Durchlass gedrückt wird.

11. Gasfallenvorrichtung nach Anspruch 10, wobei die Kappe eine gekrümmte freiliegende Oberfläche ohne einen Handgriff aufweist.

12. Gasfallenvorrichtung nach entweder Anspruch 10 oder Anspruch 11, wobei die Kappe durch ein Band (2530) mit dem Lüftungsdurchlass verbunden ist, wobei das Band einen um den Lüftungsdurchlass in Eingriff gebrachten Ring aufweist, um eine Drehung um eine Achse des Lüftungsdurchlasses zuzulassen, und wobei die Kappe zum Verschließen nur ohne Entfernen ausgelegt ist und optional die Kappe und der Lüftungsdurchlass ineinander eingreifende Merkmale (2606, 2612) aufweisen, die mit dem Drücken der Kappe in den Lüftungsdurchlass einen Schnappverschluss bereitstellen.

13. Gasfallenvorrichtung nach einem der vorhergehenden Ansprüche, wobei die Zuflussröhre einen Diffusor stromaufwärts des Umleiters umfasst, wobei der Diffusor einen Ring von Durchtrittsöffnungen um den Zuflussröhrenumfang herum umfasst.

14. Gasfallenvorrichtung nach Anspruch 13, wobei die Einlassöffnungen an einem Zusammenflussraum zusammenlaufen und die Kammer den Diffusor umfasst, der Durchtrittsöffnungen (107) um die Zuflussröhre herum für den Zufluss von Fluiden in die Kammer mit einer Querabmessungskomponente umfasst, die Einlassöffnungen (104, 105) einen Durchmesser im Bereich von 1,5 mm bis 8,0 mm aufweisen und der Abstand zwischen einem oberen Ende eines Zusammenflussraums zwischen den Öffnungen (104, 105) und den Diffusordurchtrittsöffnungen (107) nicht größer als 20 mm ist.

15. Infusionsvorrichtung, die eine Gasfallenvorrichtung nach einem der vorhergehenden Ansprüche und mit den Einlassöffnungen verbundene Infusionsleitungen und eine mit dem Auslass verbundene Infusionsleitung umfasst.

## Revendications

1. Appareil collecteur de gaz pour fluides médicaux, l'appareil comportant une chambre de piégeage de bulles (101), une entrée (103) donnant sur la chambre de piégeage de bulles avec une pluralité d'orifices d'entrée (104, 105), et une sortie avec au moins un orifice de sortie (110),
dans lequel les orifices d'entrée (104, 105) sont adaptés pour permettre au fluide s'écoulant au travers des orifices d'entrée de se mélanger lors de son entrée dans la chambre (101),
dans lequel l'appareil comporte un déflecteur d'écoulement (102) dans la chambre agencé pour dévier l'écoulement de l'entrée (103),
**caractérisé en ce que**
le déflecteur d'écoulement (102) est agencé pour dévier l'écoulement de l'entrée (103) de manière à avoir un composant de direction allant dans le sens latéral par rapport à une direction d'écoulement allant dans le sens longitudinal depuis l'entrée et à l'opposé de l'orifice de sortie et vers une surface interne de la chambre, et dans lequel le déflecteur a une forme agencée pour dévier l'écoulement distalement de l'entrée et radialement avant que l'écoulement ne passe devant le déflecteur et puisse se déplacer radialement vers l'intérieur en direction de la sortie.

2. Appareil collecteur de gaz selon la revendication 1, dans lequel l'entrée comprend une barrière d'écoulement induisant une turbulence (120), et éventuellement au moins un orifice d'entrée comprend une barrière d'écoulement induisant une turbulence (120).

3. Appareil collecteur de gaz selon la revendication 1 ou la revendication 2, dans lequel la chambre comprend une membrane hydrophile (603).

4. Appareil collecteur de gaz selon la revendication 3, dans lequel la membrane (603) est montée à une extrémité distale du déflecteur.

5. Appareil collecteur de gaz selon l'une quelconque des revendications précédentes, dans lequel au moins un orifice comprend un robinet (705) servant à fermer l'orifice entièrement ou partiellement.

6. Appareil collecteur de gaz selon la revendication 5, dans lequel une pluralité d'orifices d'entrée comporte un robinet, et l'appareil comporte un mécanisme pour l'interconnexion des robinets et leur fonctionnement synchronisé.

7. Appareil collecteur de gaz selon la revendication 5 ou la revendication 6, dans lequel l'entrée comporte un seul robinet (906) qui est monté pour ouvrir et fermer tour à tour une pluralité d'orifices d'entrée.

8. Appareil collecteur de gaz selon l'une quelconque des revendications précédentes, dans lequel l'entrée comporte un conduit commun (708, 853) pour une pluralité d'orifices (709 -706, 854), et éventuellement ledit conduit commun s'étend parallèlement à la direction allant dans le sens longitudinal.

9. Appareil collecteur de gaz selon l'une quelconque des revendications précédentes, dans lequel l'appareil comporte un orifice de ventilation (920) qui est en mesure d'être scellé, l'orifice de ventilation comportant une ouverture de ventilation (2110) et un capuchon (2101) qui est en mesure de se mettre en prise avec l'ouverture de ventilation.

10. Appareil collecteur de gaz selon la revendication 9, dans lequel le capuchon et l'ouverture de ventilation ont des éléments à mise en prise mutuelle (2104, 2105) qui sont en mesure de se mettre en prise en poussant le capuchon jusque dans l'ouverture.

11. Appareil collecteur de gaz selon la revendication 10, dans lequel le capuchon a une surface exposée courbe sans poignée.

12. Appareil collecteur de gaz selon l'une quelconque des revendications 10 ou 11, dans lequel le capuchon est relié à l'ouverture de ventilation par un ruban (2530), dans lequel le ruban a un anneau mis en prise autour de l'ouverture de ventilation pour permettre la rotation autour d'un axe de l'ouverture de ventilation, et dans lequel le capuchon est configuré à des fins de fermeture uniquement sans retrait, et éventuellement le capuchon et l'ouverture de ventilation ont des éléments à mise en prise mutuelle (2606, 2612) qui assurent un verrouillage par encliquetage lorsque le capuchon est poussé jusque dans l'ouverture de ventilation.

13. Appareil collecteur de gaz selon l'une quelconque des revendications précédentes, dans lequel le tube d'amenée comporte un diffuseur en amont du déflecteur, ledit diffuseur comportant un anneau d'ouvertures autour de la circonférence du tube d'amenée.

14. Appareil collecteur de gaz selon la revendication 13, dans lequel les orifices d'entrée fusionnent au niveau d'un espace de confluence et la chambre comporte ledit diffuseur comportant des ouvertures (107) autour du tube d'amenée pour l'amenée de fluides jusque dans la chambre avec un composant de dimension allant dans le sens latéral, les orifices d'entrée (104, 105) ont un diamètre se trouvant dans la plage allant de 1,5 mm à 8,0 mm, et la distance entre une extrémité supérieure d'un espace de confluence entre les orifices (104, 105) et les ouvertures de diffuseur (107) n'est pas supérieure à 20 mm.

15. Appareil de perfusion comportant un appareil collecteur de gaz selon l'une quelconque des revendications précédentes et des lignes de perfusion reliées aux orifices d'entrée et une ligne de perfusion reliée à la sortie.
